# EUROPEAN PATENT SPECIFICATION

(11) **EP 3 973 948 B1**
(45) Date of publication and mention of the grant of the patent: **07.01.2026**
(21) Application number: 22152960.5
(22) Date of filing: 29.07.2019
(51) Int. Cl.: A61K 8/65, A61K 8/73, A61Q 19/08

(54) **COSMETIC COMPOSITION WITH COMBINED FILLER AND SKIN REGENERATIVE EFFECT**
KOSMETISCHE ZUSAMMENSETZUNG MIT KOMBINIERTEN FÜLL- UND HAUTREGENERATIVEN EFFEKTEN
COMPOSITION COSMETIQUE AUX EFFETS COMBINES DE REMPLISSAGE ET REGENERATIFS

(43) Date of publication of application: 30.03.2022
(62) Divisional of application: 19758620.9
(73) Proprietor: Giuliani S.p.A., 20129 Milano (IT)
(72) Inventor: GIULIANI, Giammaria, 6926 Montagnola (CH); RINALDI, Fabio, 20129 MILANO (IT); SPARAVIGNA, Adele, 20159 MILANO (IT); MARZANI, Barbara, 27020 CARBONARA AL TICINO (IT)
(74) Representative: Modiano, Micaela Nadia

(56) References cited:
- EP-A1- 3 466 981
- WO-A1-2013/036568
- WO-A1-2019/211854
- WO-A2-2009/024350
- WO-A2-2018/144093
- CN-A- 108 261 341
- RINALDI FABIO ET AL: "In vitro and in vivo Evaluation on the Safety and Efficacy of a Brand-New Intracutaneous Filler with [alpha]1-R-Collagen", CLINICAL, COSMETIC AND INVESTIGATIONAL DERMATOLOGY, vol. Volume 14, 1 May 2021 (2021-05-01), pages 501 - 512, XP093043256, Retrieved from the Internet <URL:https://www.dovepress.com/getfile.php?fileID=69331> DOI: 10.2147/CCID.S295618

## Description

### FIELD OF THE INVENTION

The present invention relates to an injectable dermal filler with combined filling and regenerative effect on skin.

The present invention origins in the cosmetic and aesthetic fields.

### PRIOR ART

Human skin aging is a multifactorial, complex and chronic biological process affecting the main skin components.

Even if the skin aging mechanism is not yet fully know, recent studies have shown that the skin tends to lose the molecules responsible for binding and retaining water molecules while a reduction in the number and functionality of fibroblasts and collagen occurs.

The exposure of the skin to external factors such as solar radiation, heat or cold temperatures contributes to accelerate the aging of the skin.

Skin aging especially in the face of an individual manifests with a thinning of the epidermis and a loss of elasticity and with the appearance of wrinkles, furrows, atrophy, relaxation and loss of skin elasticity.

In the cosmetic field, preparations containing substances with moisturizing, emollient and regenerating action are commonly used to improve the skin appearance and remedy to the signs of skin aging.

However, the cosmetic effects resulting from the topical applications of these cosmetic substances is not considered fully satisfactory by the users.

For this reasons, certain techniques providing for the intradermal injection of dermo-cosmetic fillers have been developed in alternative to the topical application of cosmetic substances.

In aesthetic medicine, the dermal fillers are used to treat these signs of aging and to fill skin wrinkles or increase volumes.

Fillers are generally polymeric products injectable subcutaneously in the vicinity of wrinkles, skin furrows and in general in areas of the body where there is a sagging skin.

Once injected, they mainly carry out a mechanical action of filling the tissues, causing an action of stretching and relaxation of the skin which reduces the depth of the epidermal furrows.

The fillers available on the market can be divided into two main categories, those based on biodegradable substances and those non-biodegradable.

Non-biodegradable fillers are called permanent fillers because they are not absorbable and are generally encapsulated in fibrous tissues and are not subject to hydrolysis or phagocytosis. While exerting a prolonged action, however, permanent fillers have a higher risk of developing allergic and rejection reactions by the human body.

Typical examples of permanent fillers include liquid silicones, silicone particles in suspension, particles of acrylic hydrogel crosslinked with hyaluronic acid, calcium hydroxyapatite, polymethylmethacrylate microspheres dispersed in collagen and formulations of polyacrylamide gel.

Biodegradable fillers are called temporary because their effect wears off over time and their application needs to be renewed to maintain unchanged the filling effect. Biodegradable fillers are generally divided into two types, the intermediate duration ones and the long duration ones. The intermediate duration ones are absorbable after a few months of implantation and are made of materials subject to degradation by enzymatic hydrolysis, such as hyaluronic acid, and therefore they gradually lose the initial ability to fill and relax the tissues. Typically these polymers are biodegradable materials present in nature, with high biocompatibility and affinity with water.

However, these polymeric matrices, such as hyaluronic acid, are rapidly hydrolysed once implanted in a tissue, and the results obtained, although appreciable from an aesthetic point of view, do not last long.

In order to remedy these problems, the biodegradable polymer matrix have been modified so as to obtain polymers in crosslinked form with longer duration of action.

At present, hyaluronic acids are available on the market which are treated with crosslinking agents so as to form covalent bonds which make the resulting polymer matrix less subject to enzymatic hydrolysis and enzymatic degradation, once injected into the tissues.

Because these crosslinked polymer matrices are less soluble and less prone to degradation, they behave as inert substances that play a mere tissue filling function, without making a contribution to improve the conditions of the tissues that have led to the formation or accelerated the formation of the wrinkle.

Some long duration of action fillers are also known which are made in synthetic polymer matrices. While having the advantage of being little biodegradable and thus having a long duration of action, these fillers are not free from drawbacks as they can cause allergic reactions or skin redness.

At present, there is still a demand for cosmetic products having an effective filling action combined with cosmetic properties.

WO 2013/036568 A1 discloses cosmetic dermal fillers for aesthetic tissue augmentation, such as wrinkle treatment, with hyaluronic acid cross-linked to collagen I or collagen II.

EP 3 466 981 A1 discloses an aesthetic product which is a dermal filler with hyaluronic acid cross-linked to collagen in aqueous solution, an injection device and instructions for aesthetic treatments.

CN 108 261 341 A discloses cosmetic formulations for subcutaneous injections comprising hyaluronic acid, collagen hydrolysate or collagen peptides and amino acids such as alanine and valine.

WO 2009/024350 discloses cosmetic formulation for subcutaneous injection including filler material such as hyaluronic acid and skin active molecules such as amino acids promoting collagen production in the skin.

One of the objects of the invention therefore is to provide an injectable dermal filler combining a filling effect with a biological effect on the skin.

A further object is to provide a dermocosmetic filler that, when injected in the skin, is almost free of side effects.

A further object of the invention is to provide an effective aesthetic or cosmetic method for the treatment of skin or photo-aging without using surgical treatments of the human body.

### SUMMARY OF THE INVENTION

In accordance with a first aspect, the present invention provides an injectable dermal filler as defined in claim 1.

One of the components of the injectable dermal filler is hyaluronic acid or a salt thereof which is not in crosslinked form.

The other filler component is a recombinant type 1 Collagen protein as defined hereinbelow.

The injectable dermal filler may include peptides having biological activity whose use improves the appearance of skin which preferably are selected from AlaPro, ProGly, GlyPro and mixtures thereof. Typically, if present, the peptides having biological activity on the skin stimulate the fibroblast activity combining the eutrophic and anti-aging action with the mechanical function of the filler component.

The injectable dermal filler of the invention finds application in the cosmetic and aesthetic field and in aesthetic medicine as well.

Typically, the injectable dermal filler of the invention is designed to treat signs of skin aging and/or photo-aging in a human being.

The injectable dermal filler may be used with the following indication of use: plump up thinning lips, enhance or fill in shallow areas on the face, decrease or remove the shadow or wrinkle under the eyes caused by the lower eyelid, fill in or soften the look of recessed scars, fill in or soften static wrinkles, especially on the lower face treat Static wrinkles which usually are a result of a loss of collagen and elasticity in the skin.

Typically, the injectable dermal filler is suitable to treat or ameliorate the aspect of wrinkles especially around the mouth, thin lips, and cheeks.

The injectable dermal filler may also be used on forehead wrinkles, scars, and other areas that need extra volume for a smoother look.

The injectable dermal filler combines the mechanical function of a dermal filling with a cell regeneration activity and a delay in the skin aging process due to the sustained release of the peptides having biological activity to the tissues at the injection/implantation area.

For example, the injectable dermal filler exerts a combined filling and cosmetic effect lasting from 2 to 14 months, from 3 to 10 months, from 4 to 6 months.

Typically, the injectable dermal filler of the invention may be administered by intradermal or subcutaneous injection or implantation.

According to an aspect, a cosmetic method is provided for treating skin aging and/or photo aging comprising the injection of a cosmetically effective amount of an injectable dermal filler as defined herein.

According to certain embodiments, the cosmetic method stretches reduces, mitigates wrinkles, facial furrows, reshape, or fill the volumes of the skin.

In accordance with another aspect, a method is provided for ameliorating an aesthetic aspect of the skin and/or fill the volume of skin by or injecting in the subcutaneous layer of skin an aesthetically effective amount of an injectable dermal filler according to anyone of the embodiments disclosed herein.

The present invention is described in detail herein below by making reference to the enclosed figures.

### BRIEF DESCRIPTION OF THE DRAWINGS

Some features and advantages will become apparent from the accompanying drawings, in which:
Figs. 1A and 1B are photographic images made at times before (T0) and after 7 days (T7) from the skin treatment of a facial area of a female individual by intradermal injection of a dermal filler having the formulation of Example 1;
Fig. 2 shows a bluish area in 3D image elaboration of the facial area shown in previous Figs. 1A, 1B showing a reduction of nasolabial folds and an increase in the volume on and nearby the areas of treatment.

### DETAILED DESCRIPTION OF THE INVENTION

The present invention originates from the finding that combining a dermal filler component which is a not-crosslinked hyaluronic acid or a salt thereof with a recombinant type I collagen alfa 1 chain produced by transgenic silkworms, a synergistic cosmetic structural effect is achieved on the skin affected by skin aging, photo aging.

In particular, the invention originates from the finding that the subcutaneous or intradermal injection of a injectable dermal filler combining a dermal component with mechanical and/or filler properties with a biologically active peptide component provides an effective and prolonged antiaging action on the skin.

The prolongation of the antiaging action on the skin is due either to the extension of the filling effect and to the sustained release of the biologically active peptide component in the dermis where stimulates the production of collagen.

The application onto skin or the injection in the skin of the dermo-cosmetic composition increase the volume of the treated area determining the skin stretching and relaxation and a reduction of the depth of the epidermal corrugations.

The present invention provides either therapeutic or cosmetic uses of an injectable dermal filler as herein disclosed.

According to a first aspect thereof, the present invention therefore relates to an injectable dermal filler as defined in claim 1. Embodiments of the injectable dermal filler are defined in the appended dependent claims 2-5.

In another aspect, the use of an injectable dermal filler defined in claim 1 to increase the volume of a soft tissue, especially skin, is disclosed herein.

Typically, for this use, the subcutaneous or intradermal injection of a cosmetically effective amount of the dermal filler composition is provided.

Cosmetic uses of the dermal filler are claimed in claims 6-8.

A component of the injectable dermal filler is not-crosslinked hyaluronic acid (HA), a naturally occurring non-sulfate linear polysaccharide comprising repeating disaccharide units of d-glucuronic acid and N-acetyl-d-glucosamine linked by β-1-3 and β-1-4 glycosidic bonds. Hyaluronic acid is classified in three groups depending on its molecular weights: high molecular weight (HMWHA) is greater than 1 × 10⁶ Da, low molecular weight (LMWHA) is from 0.8 to 8 × 10⁵ Da, and oligo-HA is equal to or less than 6 × 10³ Da.

Hyaluronic acid is a primary component of the extracellular matrix of human connective tissues. It is also an important structural element in the skin.

The hyaluronic acid of the composition may be in free form or as a salt in the form of a physiologically acceptable salt such as sodium or potassium hyaluronate.

A suitable hyaluronic acid has a high molecular weight as defined above. For example, a suitable not-crosslinked hyaluronic acid may have a molecular weight greater than 1.000.000 Da, from 1.200.000, to 8.000.000 Da, of 1.500.000 to 3.500.000 Dalton.

Typically, the molecular weight of hyaluronic acid as described herein are weight average molecular weight MW, expressed in Dalton.

The hyaluronic acid molecular weight may be determined using standard methods, for example as described by Ueno et al., 1988, Chem Pharm Bull. 36, 4971-4975; Wyatt 1993, Anal Chim Acta 272: 1-40; Watt Technologies 1999 "Light scattering University Dawn Course Manual and "Dawn Eos Manual" Wyatt Technology Corp. Santa Barbara CA (USA).

The not-crosslinked hyaluronic acid of the injectable dermal filler may be in the form of a gel which may be spread on the skin or administered by subcutaneous injection.

A component of the dermal filler is a recombinant type 1 collagen α1 chain produced by transgenic silkworms. Preferably, said transgenic silkworms possess an expression cassette in its genome and expresses a recombinant protein such as recombinant type 1 collagen in its middle silk gland. The expression cassette comprises a polynucleotide having the SEQ ID NO: 1 or a part thereof as described on pages 13 and 14 of EP 1 811 027 B1 which typically is linked with a structural gene for the recombinant protein.

Typically, the recombinant type 1 collagen used herein may be obtained by the method disclosed in the European Patent EP 1 811 027 B1 especially in par. [0031], [0032] or claims 16 and 17, or in its priority application JP 2004301834 filed on 15 October 2004, especially as disclosed under par. 0040.

For example, the recombinant type 1 collagen α1 chain is obtained by a process for producing a recombinant protein comprising extracting the recombinant protein from cocoons of a transgenic silkworm which possesses (in its genome) an expression cassette for expressing the recombinant protein in silkworm middle silk gland and secrete the recombinant protein in the sericin layer of silk filaments. A suitable polynucleotide comprises a polynucleotide corresponding to a promoter region of sericin 1 gene or sericin 2 gene which is functionally linked with a polynucleotide corresponding to baculovirus homologous regions especially having the nucleotide sequence of SEQ ID NO: 1 or a part thereof as reported either in EP 1 811 027 or JP 2004301834.

According to some embodiments, the injectable dermal filler contains a recombinant type 1 collagen, especially recombinant type 1 collagen α1 chain, from 0,0001 to 5%, from 0,001 to 1,000% by weight

In certain embodiments, the injectable dermal filler as disclosed herein may be a composition in a form suitable for the application by subcutaneous or intradermal injection or by implantation in a body area, especially face, of a human being.

According to some embodiments, the injectable dermal filler contains from 0.01 to 80%, from 0.5 to 60%, from 1 to 50%, or from 10 to 40% by weight of not cross-linked HA.

According to some embodiments, the an injectable dermal filler contains from 0.001 to 20%, from 0.01 to 10%, from 0,5 to 4%, by weight of recombinant type 1 collagen α1 chain.

In certain embodiments, a further component of the injectable dermal filler may be dipeptides selected from AlaPro, ProGly, GlyPro and mixtures thereof. The mixture of the three dipeptides is preferred to achieve a combined filler and regenerative effect on the skin.

In certain embodiments, the injectable dermal filler may also contain biologically active amino acid or peptide performing a function in skin care and selected from Alanine, Valine, Glycine, Proline, OH-Proline, Aspartic acid, Lisine, Glutamic Acid, Glutamine, Asparagine, Leucine and mixtures thereof. Preferably, the amino acids Alanine, Valine, Glycine, Proline, OH-Proline, lysine are L- isomers.

Advantageously, the amino acids or peptides have a biological activity on the skin, especially on skin collagen metabolism and improve the collagen synthesis rate.

In certain embodiments the amino acids as disclosed herein maintain the skin's hydration in physiological conditions, strength the immune system thus providing an overall healthy appearance to the skin. They amino acid also protect skin from free-radical damage and activates the physiological trophism of skin thus reducing the signs of ageing.

The above biologically active amino acids and/or peptides activate the fibroblasts and are active on the intracellular components of the skin thus improving the aesthetic aspect of the skin. This cosmetic effect on the skin is complementary to the main filling effects exerted by the filling components of the injectable dermal filler.

In accordance with an embodiment the injectable dermal filler comprises cosmetically effective amounts of
i) a not crosslinked hyaluronic acid and recombinant type 1 collagen α1 chain optionally with
ii) biologically active peptides AlaPro, ProGly, GlyPro.

The injectable dermal filler herein disclosed may contain a physiologically acceptable excipient or carrier.

Optionally, said physiologically acceptable carrier is an excipient, carrier or diluent suitable for intradermal or intracutaneous/intradermal administration or topical application. A suitable carrier may be water for the subcutaneous or intradermal.

Suitable carriers in liquid form include water, preferably ultrapure or for pharmaceutical use, isotonic saline solution (0.9%), aqueous solutions with phosphate buffer (PBS), Ringer's solutions, or aqueous solutions containing one or more of carbonate, citrate, acetate, glycine and mixtures thereof.

According to some embodiments, the injectable dermal filler of the invention contains one or more pH regulating agents and/or buffer systems of the conventional type in the techniques of preparation of injectable pharmaceutical solutions.

For example, phosphate or tartrate buffers may be used to adjust the pH to physiologically compatible values.

In certain embodiments the injectable dermal filler contains a NaOH solution to have a pH from 4.5 to 8, from 6 to 7.6.

Typically, the dermal filler of the invention in injectable form is a sterile and pyrogen-free saline.

According to some embodiments, the injectable dermal filler of the invention is in the form of gel or hydrogel suitable for subcutaneous or intradermal injection.

In some embodiments, the gel or hydrogel particles have a particle size equal to or less than 700 µm. For example, from 50 to 700 µm, or from 100 to 400 µm, or from 200 to 300 µm, measured as the mass median diameter by laser diffraction technique or by microscopic analysis.

According to other embodiments, the monophasic gel is almost free from gel particles.

According to some embodiments, the composition of the invention is in the form of a water-based suspension.

According to some embodiments, the injectable dermal filler of the invention further comprises one or more physiologically acceptable excipients such as antioxidants, diluents, solvents, preservatives, bactericidal agents, stabilizers, emulsifiers, surfactants, buffers, humectants, dyes, or other excipients commonly used in cosmetic/pharmaceutical preparation techniques.

Suitable excipients are sodium chloride, sodium phosphate monobasic and or dibasic, cellulose and its derivatives such as carboxymethylcellulose. hydroxymethylcellulose, hydroxypropyl methylcellulose, methylcellulose, hydroxypropylcellulose, hydroxyethylcellulose, carboxyethylcellulose, ethylhydroxyethylcellulose, carboxymethylcellulose, cellulose acetate butyrate, cellulose acetate phthalate, and mixtures thereof.

The injectable dermal filler of the invention may further contain one or more antioxidants such sodium bisulfite, glutathione, coenzyme Q and mixtures thereof or vitamins such as vitamin B6, vitamin C.

In some embodiments, the injectable dermal filler of the invention may comprise further cosmetically active substances which can be administered by injection.

In accordance with an aspect of the invention, a cosmetic or aesthetic treatment method is provided that comprises application of a cosmetically acceptable amount of a composition as defined herein.

The cosmetic or aesthetic treatment method of the invention may be applied in the treatment of any area of the body, such as the face, the lips, the area around the eyes, breasts, buttocks, and in particular to improve the aesthetic quality of a anatomical feature.

According to certain embodiments, the injectable dermal filler of the invention is suitable for use for aesthetic purposes, in particular to increase the volume of areas of the body, for example the lips, around the eyes, or in the treatment, prevention, distension of wrinkles, skin folds or corrugations, particularly those located on the face.

In some embodiments, the cosmetic treatment method involves the intradermal or subcutaneous injection of a composition according to any one of the embodiments previously described for filling wrinkles, facial corrugations, skin depressions or scars.

In certain embodiments, the injectable dermal filler of the invention may contain a local anesthetic, such as lidocaine, tetracaine, mepivacaine, procaine, benzocaine, and mixtures thereof, analgesics such as non-steroidal anti-inflammatory drugs, antimicrobials such as antibiotics for local use and mixtures thereof.

In some embodiments, the active principles contained in the dermal filler of the present invention can be combined or mixed as active principles in intimate admixture with a suitable carrier and/or excipient according to pharmaceutical techniques.

According to some aspects of the present invention, the injectable dermal filler of the invention can be applied in the field of aesthetic medicine.

In some embodiments, the injectable dermal filler of the invention is applied in reconstructive surgery, such as in maxillofacial or aesthetic surgery, for example in plastic surgery or conservative aesthetics, for example in blepharoplasty or rhinoplasty.

In accordance with other aspects of the present invention, the injectable dermal filler of the invention can be applied in cosmetic and/or aesthetic field.

In accordance with an aspect of the invention, a method for the treatment of an area of the body of an individual for aesthetic purposes is provided comprising the application or administration to an individual of a filler or of a dermal filler according to the invention.

### Definitions

In the present document, the term "carrier" refers to an excipient, carrier, diluent or adjuvant which may be present in the composition of the invention. Any carrier and/or excipient suitable for the desired form of preparation for administration is envisaged in the uses described herein.

The term "cosmetically acceptable" as used herein means that the composition or components thereof are suitable for cosmetic use and when applied, do not cause toxicity, allergic response, redness, incompatibility, instability and similar undesired reactions.

The term "physiologically acceptable" means that the substance, when applied or administered to the human body, does not cause toxicity, allergic response, redness, incompatibility, instability and similar undesired reactions.

In general, the term "crosslinked" refers to intermolecular bonds that bind molecules or individual polymer chains such as HA or monomeric moieties thereof in a more stable structure.

Within the scope of invention, the term "monophasic gel" means a network of crosslinked viscoelastic gel comprising less than 20%, less than 10%, less than 5% by weight of gel particles.

The term biocompatible means a product or products that are physiologically acceptable and that when injected do not cause significant adverse reactions or anyway if they occur, they are of local type and are among the reactions and discomfort commonly encountered upon injection or implantation of products of known use.

The following examples are provided to illustrate the present invention.

### EXAMPLE 1

Cosmetic composition comprising an injectable dermal filler as disclosed herein having the following formulation

| INGREDIENT | AMOUNT % W/W |
|---|---|
| WATER FOR INJECTIONS | q.b |
| Sodium Hyaluronate LW P-FIL | 0,01-2,000 |
| Sodium Carboxymethylcellulose MW | 0,01-4,000 |
| Human Recombinant Type 1 Collagen | 0,001-1,000 |
| ALA-PRO | 0,001-4,00 |
| PRO-GLY | 0,001-4,00 |
| GLY-PRO | 0,001-4,00 |
| L-ALANINE | 0,01-4,00 |
| L-VALINE | 0,01-4,00 |
| L-GLYCINE | 0,01-4,00 |
| L-PROLINE | 0,01-4,00 |
| L-OH-PROLINE | 0,01-4,00 |
| ASPARTIC ACID | 0,01-4,00 |
| LISINE HCL | 0,01-4,00 |
| GLUTAMIC ACID | 0,01-4,00 |
| GLUTAMINE | 0,01-4,00 |
| ASPARAGINE | 0,01-4,00 |
| LEUCINE | 0,01-4,00 |
| VITAMIN B6 | 0,01-3,500 |
| Soda Solution 30% | q.b to pH |
| SODIUM CHLORIDE | 0,01-1,00 |
| BIPHASIC SODIUM PHOSPHATE | 0,01-1,00 |
| MONOPHASIC SODIUM PHOSPHATE | 0,01-1,00 |
| | 100,000 |

### EXAMPLE 2

Composition having the following formulation

| **COMPOSITION** | **%** |
|---|---|
| Water ppi | 91,825 |
| Ala-pro | 0,001 |
| Pro-gly | 0,001 |
| Gly-pro | 0,001 |
| Alanine | 0,130 |
| Valine | 0,170 |
| Glycine | 0,150 |
| Proline | 0,090 |
| OH-Proline | 0,090 |
| Aspartic Acid | 0,150 |
| Lisine HCl | 0,100 |
| Glutamic Acid | 0,080 |
| Glutamine | 0,080 |
| Asparagine | 0,080 |
| Leucine | 0,030 |
| Vitamin B6 | 2,000 |
| Hyaluronic Acid p-fil Lw | 2,000 |
| Sodium Carboxymethylcellulose | 3,000 |
| Recombinant Type 1 Collagen | 0,022 |
| Biphasic Sodium Phoshate Dodecahydrate | 0,090 |
| Monophasic Sodium Phosphate Dihydrate | 0,017 |
| Sodium Chloride | 0,150 |
| Sodium Hydroxide Solution 30% | q.b pH 6,50-7,50 |

The above composition is obtained by a process including the following steps:
1) weigh the ppi water needed.
2) add under stirring, respectively: Sodium Chloride, Biphasic Sodium Phoshate Dodecahydrate, Monophasic Sodium Phosphate Dihydrate. Let dissolve.
3) once dissolved, add all the amino acids in the same order of the formula.
4) dissolve the amino acids and bring the pH in a range from 6,5 to 7,5. Bring the pH around 7,10
5) add the Vitamin B and let dissolve
6) add powdered Sodium Hyaluronate LW, as defined above, till complete dispersion
7) add recombinant Collagen till dispersion
8) add sodium Carboxymethylcellulose till dispersion and swelling overnight.

### EXAMPLE 3

Composition with the following formulation

| INGREDIENT | AMOUNT % W/W |
|---|---|
| WATER FOR INJECTIONS | q.b |
| Sodium Hyaluronate LW P-FIL | 0,01-2,000 |
| Sodium Carboxymethylcellulose MW | 0,01-4,000 |
| Recombinant Type 1 Collagen | 0,001-1,000 |
| ALA-PRO | 0,001-4,00 |
| PRO-GLY | 0,001-4,00 |
| GLY-PRO | 0,001-4,00 |
| Soda Solution 30% | q.b to pH |
| SODIUM CHLORIDE | 0,01-1,00 |
| BIPHASIC SODIUM PHOSPHATE | 0,01-1,00 |
| MONOPHASIC SODIUM PHOSPHATE | 0,01-1,00 |

### EXAMPLE 4

Experimental evidence of the cosmetic effect of an injectable dermal filler.

An open, clinical trial was performed on an individual of female gender of 60 years. The individual was affected by wrinkles on the face.

Two visits were performed: basal and 1 week after the injection.

The individual was treated by intradermal (subcutaneous) injection of a composition having the formulation of Example 1.

The intradermal injection was performed at time T0 (baseline) during the basal visit, after evaluations planned by the study procedure, in the malar area (zygomatic protuberance), by bolus technique using a needle. In the alternative, a cannula for aesthetic uses may be used for injecting the composition.

The amount of tested product injected, was determined by the investigator for each subject in 3 ml. This dosage may be varied from 2 to 5 ml.

The efficacy of the cosmetic effect was evaluated in a visit made 7 days (T7) after the day of the treatment.

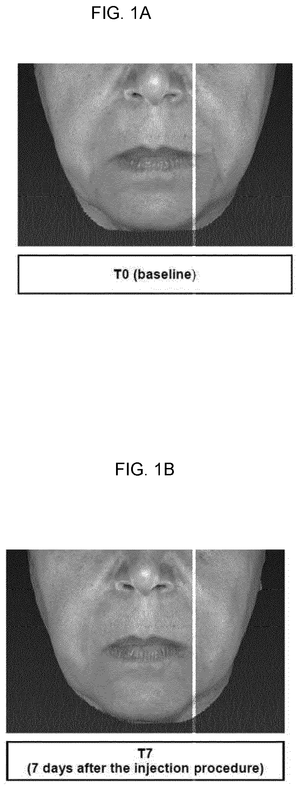

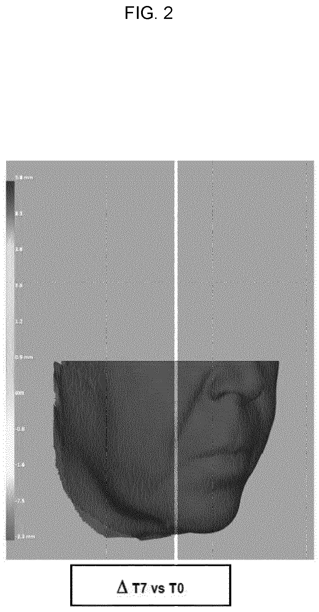

As can be seen in comparative Figures 1 and 2, after 7 days (T7) from the day T0 (baseline) of the cosmetic treatment, nasolabial folds were reduced and cheek volume increased (bluish area in 3D image elaboration). This outcome was confirmed by 3D image elaboration of the face of the individual made before and 7 days after the cosmetic treatment, as illustrated in Fig. 2.

## Claims

1. An injectable dermal filler comprising a
not-crosslinked hyaluronic acid or a salt thereof and a recombinant type I collagen α1 chain produced by transgenic silkworms.

2. The injectable dermal filler according to claim 1
comprising carboxymethylcellulose.

3. The injectable dermal filler according to anyone of claims 1-2
wherein the not-crosslinked hyaluronic acidand has a molecular weight greater than 1.000.000 Daltons.

4. The injectable dermal filler of claim 3 wherein the hyaluronic acid has a molecular weight from 1.200.000 to 8.000.000 Daltons.

5. The injectable dermal filler of claim 3 wherein the hyaluronic acid has a molecular weight from 1.500.000 to 3.500.000 Daltons.

6. Cosmetic use of a dermal filler according to anyone of claims 1-5 for improving an aesthetic aspect of an individual.

7. Cosmetic use according to claim 6 for increasing the volume or reshaping a soft tissue, especially skin of the face or neck.

8. Cosmetic use according to claim 6 for reducing the depth or ameliorating the aesthetic aspect of wrinkles, folds, corrugations, furrows, folds, in particular on the face or neck.

## Patentansprüche

1. Ein injizierbarer dermaler Füllstoff, der eine nicht quervernetzte Hyaluronsäure oder ein Salz davon und eine rekombinante Typ I-Kollagen α1-Kette umfasst, die von transgenen Seidenraupen produziert wurde.

2. Der injizierbare dermale Füllstoff gemäß Anspruch 1, der Carboxymethylcellulose umfasst.

3. Der injizierbare dermale Füllstoff gemäß einem beliebigen der Ansprüche 1-2, wobei die nicht quervernetzte Hyaluronsäure ein Molekulargewicht von mehr als 1,000,000 Dalton hat.

4. Der injizierbare dermale Füllstoff gemäß Anspruch 3, wobei die Hyaluronsäure ein Molekulargewicht von 1,200,000 bis 8,000,000 Dalton hat.

5. Der injizierbare dermale Füllstoff gemäß Anspruch 3, wobei die Hyaluronsäure ein Molekulargewicht von 1,500,000 bis 3,500,000 Dalton hat.

6. Kosmetische Verwendung eines dermalen Füllstoffs gemäß einem beliebigen der Ansprüche 1-5, um einen ästhetischen Aspekt eines Individuums zu verbessern.

7. Kosmetische Verwendung gemäß Anspruch 6, um das Volumen eines Weichgewebes zu erhöhen oder dieses neu zu formen, insbesondere Haut im Gesicht oder am Hals.

8. Kosmetische Verwendung gemäß Anspruch 6, um die Tiefe von Falten, Runzeln, Furchen zu verringern oder deren Aussehen zu verbessern, insbesondere im Gesicht oder am Hals.

## Revendications

1. Produit de remplissage dermique injectable comprenant un acide hyaluronique non réticulé ou un sel de celui-ci et une chaîne α1 de collagène de type I recombinant produit par des vers à soie transgéniques.

2. Produit de remplissage dermique injectable selon la revendication 1, comprenant de la carboxyméthylcellulose.

3. Produit de remplissage dermique injectable selon l'une quelconque des revendications 1 à 2, où l'acide hyaluronique non réticulé a un poids moléculaire supérieur à 1 000 000 daltons.

4. Produit de remplissage dermique injectable selon la revendication 3, où l'acide hyaluronique a un poids moléculaire compris entre 1 200 000 et 8 000 000 daltons.

5. Produit de remplissage dermique injectable selon la revendication 3, où l'acide hyaluronique a un poids moléculaire compris entre 1 500 000 et 3 500 000 daltons.

6. Utilisation cosmétique d'un produit de remplissage dermique selon l'une quelconque des revendications 1 à 5 pour améliorer l'aspect esthétique d'un individu.

7. Utilisation cosmétique selon la revendication 6 pour augmenter le volume ou remodeler un tissu mou, en particulier la peau du visage ou du cou.

8. Utilisation cosmétique selon la revendication 6 pour réduire la profondeur ou améliorer l'aspect esthétique des rides, plis, ondulations, sillons, en particulier sur le visage ou le cou.
